# FASCICULE DE BREVET EUROPEEN

(11) **EP 3 328 542 B1**
(45) Date de publication et mention de la délivrance du brevet: **08.09.2021**
(21) Numéro de dépôt: 16744370.4
(22) Date de dépôt: 26.07.2016
(51) Int. Cl.: B01J 31/14, B01J 31/24, C07C 2/36, C07C 2/08

(54) **PROCÉDÉ DE DIMERISATION DE L'ÉTHYLÈNE**
VERFAHREN ZUR ETHYLENDIMERISIERUNG
ETHYLENE DIMERIZATION PROCESS

(30) Priorité: 29.07.2015 FR 1557247
(43) Date de publication de la demande: 06.06.2018
(73) Titulaire: IFP Energies nouvelles, 92852 Rueil-Malmaison (FR)
(72) Inventeur: BREUIL, Pierre-Alain, 69006 Lyon (FR); CHAUMET-MARTIN, Olivia, 69530 Brignais (FR)
(74) Mandataire: IFP Energies nouvelles
(86) Numéro de dépôt international: PCT/EP2016/067756
(87) Numéro de publication internationale: WO 2017/017087

(56) Documents cités:
- FR-A5- 2 047 213
- FR-B1- 2 895 406
- US-A- 4 487 847
- US-A- 5 260 499

## Description

La présente invention concerne un procédé de dimérisation de l'éthylène en butène-1 tel que décrit par les revendications.

### Art antérieur

La transformation de l'éthylène grâce à un catalyseur homogène au nickel est étudiée depuis 1950. Cette recherche a conduit au développement et à la commercialisation de différents procédés.

La mise au point de systèmes catalytiques capables de dimériser l'éthylène en butènes passe par le choix du métal et des ligands adaptés. Parmi les systèmes existant, plusieurs systèmes catalytiques à base de nickel utilisant des ligands de type phosphine ont été développés.

Ainsi le brevet US 5,237,118 B décrit un procédé d'oligomérisation de l'éthylène mettant en oeuvre une composition catalytique comprenant un composé de nickel de degré d'oxidation zéro, un ligand phophine dans des proportions variables par rapport au composé du nickel. Ce brevet décrit par ailleurs l'utilisation d'un acide organique fluoré pour la mise en oeuvre du procédé d'oligomérisation.

US 4 487 847 A décrit un procédé de dimérisation de l'éthylène mettant en œuvre une composition catalytique comprenant un composé de nickel(II), un ligand phosphine et un acide organique.

Le brevet US 4,242,531 B décrit un procédé de dimérisation des olefines et met en oeuvre un système catalytique à base de composés de nickel chlorés de degré d'oxydation +2 et d'un activateur de type alkylaluminium halogéné. Ce brevet vise la production de butènes-2.

Le brevet GB 1 282 305 A décrit un procédé d'oligomérisation d'oléfines en présence d'une composition catalytique à base d'un métal du groupe VIII, d'un donneur d'électron tel que la phosphine et d'un composé d'aluminium. Le ratio molaire entre la phosphine et le métal dans la composition est compris entre 0,5 et 18. Cependant le brevet GB 1 282 305 A décrit un procédé mis en oeuvre à des température inférieures à 20°C et de préférence inférieures à 0°C.

Le brevet FR 1,547,921 décrit un procédé d'oligomérisation d'oléfines avec une composition catalytique à base d'halogénure de nickel et de phosphine qui nécessite une réduction préalable de la composition en vue de préparer le catalyseur actif. Les rendements en butènes sont de l'ordre de 63% C4 dont 3% de butènes-1.

Le brevet FR 1, 588,162 décrit un procédé de dimérisation d'oléfines entre C2 et C4 mettant en œuvre un système catalytique comprenant un composé du nickel et une phosphine et en particulier des halogénures d'alkyles avec des rendements en butènes de l'ordre de 80%. Le ratio molaire entre la phosphine et le métal dans la composition est compris entre 1 et 5, cependant ce brevet vise la production de butènes-2.

L'objectif de l'invention est de proposer un nouveau procédé de dimérisation de l'éthylène en butène-1 plus performant en termes de rendement et de sélectivité pour la production de butène-1.

La demanderesse dans ses recherches a mis au point un nouveau procédé mettant en œuvre une composition catalytique comprenant un précurseur de nickel de degré d'oxydation (+II), au moins un ligand phosphine, éventuellement une base de Lewis, et au moins un agent activateur, ledit procédé étant caractérisé en ce qu'il est réalisé à une température comprise entre une valeur supérieure à 20 et +250°C. Il a été constaté de manière surprenante que le procédé selon l'invention présente un bon couple rendement/sélectivité catalytique dans la production sélective de butène-1.

### Description détaillée de l'invention

La composition catalytique mise en œuvre dans le procédé de l'invention comprend :
- au moins un précurseur de nickel de degré d'oxydation (+II),
- au moins un ligand phosphine de formule PR¹R²R³ dans lequel les groupements R¹, R² et R³, identiques ou différents entre eux, liés ou non entre eux, sont choisis
   - parmi les groupements aromatiques substitués ou non et contenant ou non des hétéroéléments,
   - et/ou parmi les groupements hydrocarbyles cycliques ou non, substitués ou non et contenant ou non des hétéroéléments,
- et au moins un agent activateur choisi dans le groupe formé par les composés chlorés et bromés d'hydrocarbylaluminium, pris seuls ou en mélange,
ladite composition présentant un rapport molaire du ligand phosphine sur le précurseur de nickel compris entre 5 et 30 et un rapport molaire de l'agent activateur sur le ligand phosphine supérieur ou égal à 1, de préférence supérieur ou égal à 1,5, de préférence supérieur ou égal à 2, le procédé étant mis en œuvre à une température comprise entre 45°C et +250°C.

Avantageusement selon l'invention, la composition catalytique comprend au moins un ligand phosphine de formule PR¹R²R³ dans lequel les groupements R¹, R² et R³ sont identiques entre eux.

Les groupements aromatiques R¹, R² et R³ du ligand phosphine PR¹R²R³ sont de préférence choisis dans le groupe formé par les groupements phényle, o-tolyle, m-tolyle, p-tolyle, mésityle, 3,5-diméthylphényle, 4-n-butylphényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-di-tert-butyl-4-méthoxyphényle, 4-chlorophényle, 3,5-di(trifluorométhyl)phényle, benzyle, naphtyle, bisnaphtyle, pyridyle, bisphényle, furanyle, thiophényle.

Les groupements hydrocarbyles R¹, R² et R³ du ligand phosphine PR¹R²R³ comprennent avantageusement 1 à 20 atomes de carbone, de préférence 2 à 15 atomes de carbone, de manière préférée entre 3 et 10 atomes de carbone. De préférence, les groupements hydrocarbyles R¹, R² et R³ du ligand phosphine PR¹R²R³ sont choisis dans le groupe formé par les groupements méthyle, éthyle, propyle, isopropyle, *n*-butyle, *tert*-butyle, cyclopentyle, cyclohexyle, benzyle, adamantyle.

La composition catalytique utilisée selon l'invention peut également comprendre une base de Lewis. Au sens de la présente invention, on entend par « base de Lewis », toute entité chimique dont un constituant possède un doublet ou plus d'électrons libres ou non liants. Les bases de Lewis selon l'invention correspondent en particulier à tout ligand comprenant un atome d'oxygène ou d'azote possédant un doublet d'électrons libres ou non liants, ou une double liaison π capable de former avec le nickel une coordination de type η².

Ladite base de Lewis est de préférence choisie parmi le diéthyléther, le méthyle tert-butyléther, le tétrahydrofurane, le 1,4-dioxane, l'isoxazole, la pyridine, la pyrazine, et la pyrimidine.

Selon l'invention, l'agent activateur mis en œuvre dans la composition catalytique est choisi dans le groupe formé par les composés chlorés et bromés d'hydrocarbylaluminium pris seuls ou en mélange.

De manière avantageuse, ledit agent activateur est choisi dans le groupe formé par le dichlorure de méthylaluminium (MeAlCl₂), le dichlorure d'éthylaluminium (EtAlCl₂), le sesquichlorure d'éthylaluminium (Et₃Al₂Cl₃), le chlorure de diéthylaluminium (Et₂AlCl), le chlorure de diisobutylaluminium (*i*Bu₂AlCl), le dichlorure d'isobutylaluminium (*i*BuAlCl₂), pris seuls ou en mélange.

Avantageusement, le rapport molaire du ligand phosphine sur le précurseur de nickel est compris entre 5 et 25, de préférence entre 5 et 20, de manière plus préférée entre 5 et 15. De préférence, ce rapport molaire est compris entre 6 et 30, de préférence entre 6 et 25, de manière plus préférée entre 6 et 20, de manière encore plus préférée entre 6 et 15, et de manière encore plus préférentielle entre 7 et 12.

Avantageusement, le rapport molaire de l'agent activateur sur le ligand phosphine est de préférence supérieur ou égal à 1,5, de préférence supérieur ou égal à 2.

Le rapport molaire de l'agent activateur sur le précurseur de nickel est de préférence supérieur ou égal à 5, de manière plus préférée supérieur ou égal à 6, et de préférence inférieur ou égal à 30, de préférence inférieur ou égal à 25, de manière plus préférée inférieur ou égal à 20.

Les rapports molaires cités dans la présente invention notamment par rapport au précurseur de nickel sont entendus et exprimés par rapport au nombre de moles de nickel apportées dans la composition catalytique.

Les compositions utilisées dans le procédé selon l'invention peuvent également éventuellement comprendre un solvant. On peut utiliser un solvant choisi parmi les solvants organiques et en particulier parmi les éthers, les alcools, et les hydrocarbures saturés, insaturés, aromatiques ou non, cycliques ou non. De préférence, le solvant est choisi parmi l'hexane, le cyclohexane, le méthylecyclohexane, l'heptane, le butane ou l'isobutane ou toute autre coupe hydrocarbure ayant des points d'ébullition supérieurs à 70°C, de préférence compris entre 70°C et 200°C et de préférence compris entre 90°C et 180°C, les monooléfines ou dioléfines comportant de préférence 4 à 20 atomes de carbone, le cycloocta-1,5-diène, le benzène, l'ortho-xylène, le mésitylène, l'éthylbenzène, le méthanol, l'éthanol, purs ou en mélange, et les liquides ioniques. Dans le cas où le solvant est un liquide ionique, il est avantageusement choisi parmi les liquides ioniques décrits dans les brevets US 6, 951,831 B2 et FR 2895406 B1. De manière préférée, le solvant est choisi parmi l'hexane, le cyclohexane, le méthylecyclohexane, l'heptane, le butane ou l'isobutane ou toute autre coupe hydrocarbure non aromatique ayant des points d'ébullition supérieurs à 70°C, de préférence compris entre 70°C et 200°C et de préférence compris entre 90°C et 180°C.

Le précurseur de nickel de la composition utilisée dans le procédé selon l'invention est de degré d'oxydation +II. Il est de préférence choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate de nickel, les phénates de nickel(II), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

Avantageusement, le précurseur de nickel ne comprend pas de composés halogénés. De préférence, le précurseur de nickel est choisi parmi le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate de nickel, les phénates de nickel(II), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

Les compositions décrites ci-avant sont utilisées dans un procédé de dimérisation de l'éthylène en butène-1.

Le procédé de dimérisation selon l'invention peut opérer en présence d'un solvant. Dans ce cas le solvant peut être choisi parmi les solvants organiques et de préférence parmi les hydrocarbures saturés, insaturés, cycliques ou non. En particulier, ledit solvant est choisi parmi l'hexane, le cyclohexane, le méthylecyclohexane, l'heptane, le butane ou l'isobutane, les monooléfines ou dioléfines comportant de préférence 4 à 20 atomes de carbone, purs ou en mélange ou toute autre coupe hydrocarbure non aromatique ayant des points d'ébullition supérieurs à 70°C, de préférence compris entre 70°C et 200°C et de préférence compris entre 90°C et 180°C. Dans une variante préférée, au moins une partie des produits à l'issu du procédé sont recyclés comme solvant de procédé dans le procédé de dimérisation selon l'invention.

Le solvant du procédé peut également être choisi parmi les liquides ioniques. Dans le cas où ledit solvant de réaction est un liquide ionique, il est avantageusement choisi parmi les liquides ioniques décrits dans les brevets US 6, 951,831 B2 et FR 2895406 B1.

Avantageusement, les différents composants de la composition catalytique, notamment le précurseur de nickel de degré d'oxydation (+II), le ligand phosphine et l'agent activateur sont injectés directement dans le procédé de dimérisation selon l'invention, c'est-à-dire sans période d'activation. De préférence, chaque composant est injecté séparément dans le procédé de dimérisation de l'éthylène selon l'invention. Dans une mise en œuvre particulière, on injecte séparément dans le procédé de dimérisation de l'éthylène d'une part un mélange du précurseur de nickel(II) et du ligand phosphine et d'autre part l'agent activateur.

Les oléfines utilisées comme charge peuvent être utilisées seuls ou diluées avantageusement par un ou plusieurs alcane(s) ou toute autre coupe pétrolière, tels qu'on les trouve dans des « coupes » issues des procédés de raffinage du pétrole ou de la pétrochimie, comme le craquage catalytique ou le craquage à la vapeur.

L'oléfine utilisée comme charge dans le procédé de dimérisation est l'éthylène.

La concentration du nickel dans la solution catalytique, à savoir dans le procédé selon l'invention, est avantageusement comprise entre 1.10⁻⁸ et 1 mol/L, et de préférence entre 1.10⁻⁶ et 1.10⁻² mol/L.

Le procédé de dimérisation de l'éthylène en butène-1 opère avantageusement à une pression totale comprise entre la pression atmosphérique et 20 MPa, de préférence entre 0,1 et 8 MPa, de préférence entre 3 et 8 MPa. La température du procédé de dimérisation de l'éthylène en butène-1 est comprise entre 45 et 250°C, de manière encore plus préférée entre 45 et 70°C. Il a été trouvé de manière surprenante que le procédé de dimérisation de l'éthylène en butène-1 opérée dans les conditions selon l'invention et mis en œuvre avec la composition décrite présente l'avantage de produire du butène-1 avec un bon couple rendement/sélectivité. Avantageusement, le procédé selon l'invention permet d'obtenir une coupe butènes (C4) dans un rendement d'au moins 91% et une sélectivité en butène-1 (1-C4) d'au moins 62%.

La chaleur engendrée par la réaction peut être éliminée par tous les moyens connus de l'homme du métier.

Le procédé de dimérisation peut être conduit en système fermé, en système semi-ouvert ou en continu, avec un ou plusieurs étages de réaction. Une vigoureuse agitation est avantageusement mise en œuvre pour assurer un bon contact entre le ou les réactifs et le système catalytique.

Le procédé de dimérisation peut être mis en œuvre en discontinu. Dans ce cas, un volume choisi de la solution comprenant la composition utilisée dans le procédé de l'invention est introduit dans un réacteur de préférence muni des dispositifs habituels d'agitation, de chauffage et de refroidissement.

Le procédé de dimérisation peut également être mis en œuvre en continu. Dans ce cas, les composants de la composition selon l'invention sont injectés dans un réacteur dans lequel réagit l'oléfine, notamment l'éthylène, de préférence avec un contrôle de la température.

La composition catalytique utilisée dans le procédé de l'invention est détruite par tout moyen habituel connu par l'homme du métier, puis les produits de la réaction ainsi que le solvant sont séparés, par exemple par distillation. L'oléfine, notamment l'éthylène qui n'a pas été transformée peut être recyclée dans le réacteur.

Le procédé selon l'invention peut être mis en œuvre dans un réacteur à un ou plusieurs étages de réaction en série, la charge oléfinique et/ou les composants de la composition catalytique étant introduits en continu, soit dans le premier étage, soit dans le premier et un autre quelconque des étages. A la sortie du réacteur, la composition catalytique peut être désactivée, par exemple par injection d'une amine diluée ou non et/ou d'une solution aqueuse basique et/ou d'une solution aqueuse acide. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools, comme composants pour l'industrie chimique, pharmaceutique ou la parfumerie et/ou comme charges dans un procédé de métathèse pour la synthèse de propylène et/ou comme charge d'un procédé assurant la production de butadiène via une déshydrogénation oxydante ou via une étape de catalyse métallique par exemple.

Les exemples suivants illustrent l'invention sans en limiter la portée.

### EXEMPLES :

### Mise en œuvre du test catalytique :

Le réacteur est préalablement séché sous vide et mis sous atmosphère d'éthylène. 93 mL de n-heptane sont introduits dans le réacteur sous atmosphère d'éthylène. 6 mL d'une solution contenant le précurseur de nickel Ni(2-éthylhexanoate)₂ (noté Ni(2-EH)₂, 5 ou 10 µmol et la phosphine tri-n-butylphosphine ou P(nBu)₃, tricyclohexylphosphine ou PCy₃ ou tri-isopropylphosphine ou P(iPr)₃ (10, 20, 50 ou 100 µmol) sont ensuite introduits dans le réacteur. Entre 1 et 2 g d'éthylène sont alors solubilisés dans le réacteur, l'agitation est lancée et la température programmée à 40°C. Après dégazage du réacteur, la température est programmée à 45°C (température de test). 1 mL d'une solution de dichlorure d'éthylaluminium (75 ou 150 µmol) sont ensuite introduits. Le réacteur est mis à la pression de test (2 MPa). La consommation d'éthylène est suivie jusqu'à l'introduction de 50 g d'éthylène. L'alimentation en éthylène est alors coupée. La phase gaz est quantifiée et qualifiée par chromatographie en phase gaz (GC), la phase liquide est pesée, neutralisée et qualifiée par GC.

### Test catalytiques

### Exemples 1-2 : Evaluation du ligand tri-n-butylphosphine P(nBu)₃

| Entrée | Précurseur Catalytique (Ni) | Ligand | Ratio molaire Ligand/Ni | Temps (min) | Masse C₂H₄ cons. (g) | % C4** | % C6 | % C8⁺ | % 1-C4*** |
|---|---|---|---|---|---|---|---|---|---|
| 1* | Ni(2-EH)₂ | P(*n*Bu)₃ | 2 | 10 | 40 | 89 | 10 | 1 | 47 |
| 2 | Ni(2-EH)₂ | P(*n*Bu)₃ | 10 | 30 | 10 | 98 | 2 | 0 | 62 |

| | | | | | | | | | |
|---|---|---|---|---|---|---|---|---|---|
| n_{Ni} = 5 µmol, 15 éq. EtAlCl₂, 45°C, 2 MPa, 100 mL n-heptane. * Exemple comparatif. cons.= consommé. ** rendement en C4 correspondant pourcentage poids de la coupe C4 formé dans les produits, *** pourcentage de 1-C4 dans la coupe C4. | | | | | | | | | |

### Exemples 3-7 : Evaluation du ligand tricyclohexylphosphine (PCy₃)

| Entrée | Précurseur catalytique | Ligand. | Ratio molaire Ligand/Ni | Temps (min) | Masse C₂H₄ cons. (g) | % C4** | % C6 | % C8⁺ | % 1-C4*** |
|---|---|---|---|---|---|---|---|---|---|
| 3* | Ni(2-EH)₂ | PCy₃ | 2 | 10 | 16 | 91 | 9 | 0 | 59 |
| 4*^{a} | Ni(2-EH)₂ | PCy₃ | 10 | 60 | < 1 | non déterminés | | | |
| 5*^{b} | Ni(2-EH)₂ | PCy₃ | 10 | 60 | < 1 | non déterminés | | | |
| 6 | Ni(2-EH)₂ | PCy₃ | 10 | 40 | 20 | 96 | 4 | 0 | 97 |
| 7^{c} | Ni(2-EH)₂ | PCy₃ | 10 | 10 | 41 | 93 | 7 | 0 | 81 |

| | | | | | | | | | |
|---|---|---|---|---|---|---|---|---|---|
| n_{Ni} = 5 µmol, 15 éq. EtAlCl₂, 45°C, 2 MPa, 100 mL n-heptane. * Exemple comparatif. ^{a} Test réalisé à 20°C dans le chlorobenzène comme solvant de réaction. ^{b} Test réalisé à 20°C dans le n-heptane comme solvant de réaction. ^{c}n_{Ni} = 10 µmol. cons.= consommé. ** rendement en C4 correspondant pourcentage poids de la coupe C4 formé dans les produits, *** pourcentage de 1-C4 dans la coupe C4. | | | | | | | | | |

### Exemples 8-9 : Evaluation du ligand tri-isopropylphosphine P(iPr)₃

| Entrée | Précurseur Catalytique | Ligand | Ratio molaire Ligand/Ni | Temps (min) | Masse C₂H₄ cons. (g) | % C4** | % C6 | % C8⁺ | % 1-C4*** |
|---|---|---|---|---|---|---|---|---|---|
| 8* | Ni(2-EH)₂ | P(iPr)₃ | 2 | 15 | 37 | 89 | 10 | 1 | 56 |
| 9 | Ni(2-EH)₂ | P(iPr)₃ | 10 | 16 | 36 | 94 | 6 | 0 | 89 |

| | | | | | | | | | |
|---|---|---|---|---|---|---|---|---|---|
| n_{Ni} = 10 µmol, 15 éq. EtAlCl₂, 45°C, 2 MPa, 100 mL n-heptane. * Exemple comparatif. cons.= consommé. ** rendement en C4 correspondant pourcentage poids de la coupe C4 formé dans les produits, *** pourcentage de 1-C4 dans la coupe C4. | | | | | | | | | |

On constate que les compositions catalytiques selon le procédé de l'invention (entrées 2, 6, 7 et 9) permettent l'obtention d'une coupe butènes (C4) dans un rendement d'au moins 93% et une sélectivité en butène-1 (1-C4) d'au moins 62% comparés aux compositions catalytiques non conformes à l'invention (entrées 1, 3, 8) qui présentent un rendement de 91% maximum en C4 et une sélectivité en butènes-1 (1-C4) comprise entre 47 et 59%.

## Revendications

1. Procédé de dimérisation de l'éthylène en butène-1 comprenant la mise en contact de l'éthylène avec une composition catalytique comprenant :
- au moins un précurseur de nickel de degré d'oxydation (+II),
- au moins un ligand phosphine de formule PR¹R²R³ dans lequel les groupements R¹, R² et R³, identiques ou différents entre eux, liés ou non entre eux, sont choisis
- parmi les groupements aromatiques substitués ou non et contenant ou non des hétéroéléments,
- et/ou parmi les groupements hydrocarbyles cycliques ou non, substitués ou non et contenant ou non des hétéroéléments,
- et au moins un agent activateur choisi dans le groupe formé par les composés chlorés et bromés d'hydrocarbylaluminium, pris seuls ou en mélange,
ladite composition présentant un rapport molaire du ligand phosphine sur le précurseur de nickel compris entre 5 et 30 et un rapport molaire de l'agent activateur sur le ligand phosphine supérieur ou égal à 1, le procédé étant mis en œuvre à une température comprise entre 45°C et 250°C.

2. Procédé selon la revendication 1 dans laquelle les groupements R¹, R² et R³ dudit ligand phosphine sont identiques.

3. Procédé selon l'une des revendications 1 ou 2 dans laquelle les groupements aromatiques R¹, R² et R³ du ligand phosphine PR¹R²R³ sont choisis dans le groupe formé par les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-di-tert-butyl-4-méthoxyphényle, 4-chlorophényle, 3,5-di(trifluorométhyl)phényle, benzyle, naphthyle, bisnaphtyle, pyridyle, bisphényle, furanyle, thiophényle.

4. Procédé selon l'une des revendications précédentes dans laquelle les groupements hydrocarbyles R¹, R² et R³ du ligand phosphine PR¹R²R³ comprennent 1 à 20 atomes de carbone.

5. Procédé selon la revendication 4 dans laquelle les groupements hydrocarbyles R¹, R² et R³ du ligand phosphine PR¹R²R³ sont choisis dans le groupe formé par les groupements méthyle, éthyle, propyle, isopropyle, n-butyle, tert-butyle, cyclopentyle, cyclohexyle, benzyle, adamantyle.

6. Procédé selon l'une des revendications précédentes dans laquelle le rapport molaire du ligand phosphine sur le précurseur de nickel est compris entre 5 et 25.

7. Procédé selon l'une des revendications précédentes dans laquelle ladite composition comprend également une base de Lewis.

8. Procédé selon la revendication 7 dans laquelle la base de Lewis est choisie parmi le diéthyléther, le méthyle tert-butyléther, le tétrahydrofurane, le 1,4-dioxane, l'isoxazole, la pyridine, la pyrazine, et la pyrimidine.

9. Procédé selon l'une des revendications précédentes dans laquelle l'agent activateur est choisi dans le groupe formé par le dichlorure de méthylaluminium (MeAlCl₂), le dichlorure d'éthylaluminium (EtAlCl₂), le sesquichlorure d'éthylaluminium (Et₃Al₂Cl₃), le chlorure de diéthylaluminium (Et₂AlCl), le chlorure de diisobutylaluminium (*i*Bu₂AlCl), le dichlorure d'isobutylaluminium (*i*BuAlCl₂), pris seuls ou en mélange.

10. Procédé selon l'une des revendications précédentes dans laquelle le précurseur de nickel est choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

11. Procédé selon l'une des revendications 1 à 10 dans laquelle le précurseur de nickel est choisi parmi le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

12. Procédé selon l'une des revendications 1 à 11 conduit en système fermé, en système semi-ouvert, en continu ou en discontinu.

## Patentansprüche

1. Verfahren zur Dimerisierung von Ethylen zu 1-Buten, umfassend das Inkontaktbringen von Ethylen mit einer katalytischen Zusammensetzung, umfassend:
- mindestens eine Nickelvorstufe in der Oxidationsstufe (+II),
- mindestens einen Phosphinliganden der Formel PR¹R²R³, wobei die Gruppen R¹, R² und R³, die gleich oder voneinander verschieden sind und gegebenenfalls aneinander gebunden sind,
- aus gegebenenfalls substituierten und gegebenenfalls Heteroelemente enthaltenden aromatischen Gruppen
- und/oder aus cyclischen oder acyclischen, gegebenenfalls substituierten und gegebenenfalls Heteroelemente enthaltenden Hydrocarbylgruppen ausgewählt sind,
- und mindestens ein Aktivierungsmittel, ausgewählt aus der Gruppe bestehend aus chlorierten und bromierten Hydrocarbylaluminiumverbindungen alleine oder als Mischung,
wobei die Zusammensetzung ein Molverhältnis von Phosphinligand zu Nickelvorstufe zwischen 5 und 30 und ein Molverhältnis von Aktivierungsmittel zu Phosphinligand größer als oder gleich 1 aufweist, wobei das Verfahren bei einer Temperatur zwischen 45 °C und 250 °C durchgeführt wird.

2. Verfahren nach Anspruch 1, wobei die Gruppen R¹, R² und R³ des Phosphinliganden gleich sind.

3. Verfahren nach Anspruch 1 oder 2, wobei die aromatischen Gruppen R¹, R² und R³ des Phosphinliganden PR¹R²R³ aus der Gruppe bestehend aus Phenyl-, o-Tolyl-, m-Tolyl-, p-Tolyl-, Mesityl-, 3,5-Dimethylphenyl-, 4-n-Butylphenyl-, 4-Methoxyphenyl-, 2-Methoxyphenyl-, 3-Methoxyphenyl-, 4-Methoxyphenyl-, 2-Isopropoxyphenyl-, 4-Methoxy-3,5-dimethylphenyl-, 3,5-Di-tert-butyl-4-methoxyphenyl-, 4-Chlorphenyl-, 3,5-Di(trifluormethyl)phenyl-, Benzyl-, Naphthyl-, Bisnaphthyl-, Pyridyl-, Bisphenyl-, Furanyl- und Thiophenylgruppen ausgewählt sind.

4. Verfahren nach einem der vorhergehenden Ansprüche, wobei die Hydrocarbylgruppen R¹, R² und R³ des Phosphinliganden PR¹R²R³ 1 bis 20 Kohlenstoffatome umfassen.

5. Verfahren nach Anspruch 4, wobei die Hydrocarbylgruppen R¹, R² und R³ des Phosphinliganden PR¹R²R³ aus der Gruppe bestehend aus Methyl-, Ethyl-, Propyl-, Isopropyl-, n-Butyl-, tert-Butyl-, Cyclopentyl-, Cyclohexyl-, Benzyl- und Adamantylgruppen ausgewählt sind.

6. Verfahren nach einem der vorhergehenden Ansprüche, wobei das Molverhältnis von Phosphinligand zu Nickelvorstufe zwischen 5 und 25 liegt.

7. Verfahren nach einem der vorhergehenden Ansprüche, wobei die Zusammensetzung außerdem eine Lewis-Base umfasst.

8. Verfahren nach Anspruch 7, wobei die Lewis-Base aus Diethylether, Methyl-tert-butylether, Tetrahydrofuran, 1,4-Dioxan, Isoxazol, Pyridin, Pyrazin und Pyrimidin ausgewählt ist.

9. Verfahren nach einem der vorhergehenden Ansprüche, wobei das Aktivierungsmittel aus der Gruppe bestehend aus Methylaluminiumdichlorid (MeAlCl₂), Ethylaluminiumdichlorid (EtAlCl₂), Ethylaluminiumsesquichlorid (Et₃Al₂Cl₃), Diethylaluminiumchlorid (Et₂AlCl), Diisobutylaluminiumchlorid (*i*Bu₂AlCl) und Isobutylaluminiumdichlorid (*i*BuAlCl₂) alleine oder als Mischung ausgewählt ist.

10. Verfahren nach einem der vorhergehenden Ansprüche, wobei die Nickelvorstufe aus Nickel(II)-chlorid, Nickel(II)-chlorid(dimethoxyethan), Nickel(II)-bromid, Nickel(II)-bromid(dimethoxyethan), Nickel(II)-fluorid, Nickel(II)-iodid, Nickel(II)-sulfat, Nickel(II)-carbonat, Nickel(II)-dimethyl-glyoxim, Nickel(II)-hydroxid, Nickel(II)-hydroxyacetat, Nickel(II)-oxalat, Nickel(II)-carboxylaten, n-Allylnickel(II)-chlorid, n-Allylnickel(II)-bromid, Methallylnickel(II)-chlorid-Dimer, η³-Allylnickel(II)-hexafluorophosphat, η³-Methallylnickel(II)-hexafluorophosphat und 1,5-Cyclooctadienylnickel(II) alleine oder als Mischung ausgewählt ist.

11. Verfahren nach einem der Ansprüche 1 bis 10, wobei die Nickelvorstufe aus Nickel(II)-sulfat, Nickel(II)-carbonat, Nickel(II)-dimethylglyoxim, Nickel(II)-hydroxid, Nickel(II)-hydroxyacetat, Nickel(II)-oxalat, Nickel(II)-carboxylaten, n-Allylnickel(II)-chlorid, π-Allylnickel(II)-bromid, Methallylnickel(II)-chlorid-Dimer, η³-Allylnickel-(II)-hexafluorophosphat, η³-Methallylnickel(II)-hexafluorophosphat und 1,5-Cyclooctadienylnickel-(II) alleine oder als Mischung ausgewählt ist.

12. Verfahren nach einem der Ansprüche 1 bis 11, das in einem geschlossenen System, in einem halbgeschlossenen System, kontinuierlich oder diskontinuierlich durchgeführt wird.

## Claims

1. Process for the dimerization of ethylene to 1-butene, comprising bringing ethylene into contact with a catalytic composition comprising:
- at least one nickel precursor with an oxidation state of (+II),
- at least one phosphine ligand with formula PR¹R²R³, in which the R¹, R² and R³ groups, which may be identical or different, and which may optionally be bonded together, are chosen
- from substituted or unsubstituted aromatic groups optionally containing heteroelements,
- and/or from cyclic or non-cyclic, substituted or unsubstituted hydrocarbyl groups optionally containing heteroelements,
- and at least one activating agent chosen from the group formed by chlorinated and brominated hydrocarbylaluminium compounds, used alone or as a mixture,
said composition having a molar ratio of the phosphine ligand to the nickel precursor of between 5 and 30 and a molar ratio of the activating agent to the phosphine ligand of greater than or equal to 1,
the process being carried out at a temperature between 45°C and 250°C.

2. Process according to Claim 1, in which the R¹, R² and R³ groups of said phosphine ligand are identical.

3. Process according to either one of Claims 1 and 2, in which the aromatic R¹, R² and R³ groups of the phosphine ligand PR¹R²R³ are chosen from the group formed by phenyl, o-tolyl, m-tolyl, p-tolyl, mesityl, 3,5-dimethylphenyl, 4-n-butylphenyl, 4-methoxyphenyl, 2-methoxyphenyl, 3-methoxyphenyl, 4-methoxyphenyl, 2-isopropoxyphenyl, 4-methoxy-3,5-dimethylphenyl, 3,5-di-tert-butyl-4-methoxyphenyl, 4-chlorophenyl, 3,5-di(trifluoromethyl)-phenyl, benzyl, naphthyl, bisnaphthyl, pyridyl, bisphenyl, furanyl and thiophenyl groups.

4. Process according to one of the preceding claims, in which the hydrocarbyl R¹, R² and R³ groups of the phosphine ligand PR¹R²R³ comprise 1 to 20 carbon atoms.

5. Process according to Claim 4, in which the hydrocarbyl R¹, R² and R³ groups of the phosphine ligand PR¹R²R³ are chosen from the group formed by methyl, ethyl, propyl, isopropyl, n-butyl, tert-butyl, cyclopentyl, cyclohexyl, benzyl and adamantyl groups.

6. Process according to one of the preceding claims, in which the molar ratio of the phosphine ligand to the nickel precursor is between 5 and 25.

7. Process according to one of the preceding claims, in which said composition also comprises a Lewis base.

8. Process according to Claim 7, in which the Lewis base is selected from diethyl ether, methyl tert-butyl ether, tetrahydrofuran, 1,4-dioxane, isoxazole, pyridine, pyrazine and pyrimidine.

9. Process according to one of the preceding claims, in which the activating agent is chosen from the group formed by methylaluminium dichloride (MeAlCl₂), ethylaluminium dichloride (EtAlCl₂), ethylaluminium sesquichloride (Et₃Al₂Cl₃), diethylaluminium chloride (Et₂AlCl), diisobutylaluminium chloride (*i*Bu₂AlCl), and isobutylaluminium dichloride (*i*BuAlCl₂), taken alone or as a mixture.

10. Process according to one of the preceding claims, in which the nickel precursor is chosen from nickel(II) chloride, nickel(II) chloride (dimethoxyethane), nickel(II) bromide, nickel(II) bromide (dimethoxyethane), nickel(II) fluoride, nickel(II) iodide, nickel(II) sulfate, nickel(II) carbonate, nickel(II) dimethylglyoxime, nickel(II) hydroxide, nickel(II) hydroxyacetate, nickel(II) oxalate, nickel(II) carboxylates, π-allylnickel(II) chloride, π-allylnickel(II) bromide, methallylnickel(II) chloride dimer, η³-allylnickel(II) hexafluorophosphate, η³-methallylnickel(II) hexafluorophosphate and 1,5-cyclooctadienylnickel(II), in their hydrated or non-hydrated form, taken alone or as a mixture.

11. Process according to one of Claims 1 to 10, in which the nickel precursor is chosen from nickel(II) sulfate, nickel(II) carbonate, nickel(II) dimethylglyoxime, nickel(II) hydroxide, nickel(II) hydroxyacetate, nickel(II) oxalate, nickel(II) carboxylates, π-allylnickel(II) chloride, π-allylnickel(II) bromide, methallylnickel(II) chloride dimer, η³-allylnickel(II) hexafluorophosphate, η³-methallylnickel(II) hexafluorophosphate and 1,5-cyclooctadienylnickel(II), in their hydrated or non-hydrated form, taken alone or as a mixture.

12. Process according to one of Claims 1 to 11, carried out in a closed system, in a semi-open system, continuously or batchwise.
